# EUROPEAN PATENT SPECIFICATION

(11) **EP 3 621 466 B1**
(45) Date of publication and mention of the grant of the patent: **01.12.2021**
(21) Application number: 18725595.5
(22) Date of filing: 09.05.2018
(51) Int. Cl.: A24F 40/51, A24F 40/60, A61M 15/00, A61M 15/06, A61M 11/04, A61M 16/00

(54) **VAPOUR PROVISION SYSTEMS**
DAMPFBEREITSTELLUNGSSYSTEME
SYSTÈMES DE FOURNITURE DE VAPEUR

(30) Priority: 12.05.2017 GB 201707627
(43) Date of publication of application: 18.03.2020
(73) Proprietor: Nicoventures Trading Limited, London WC2R 3LA (GB)
(72) Inventor: HEPWORTH, Richard, London WC2R 3LA (GB); DICKENS, Colin, London WC2R 3LA (GB); MOLONEY, Patrick, London WC2R 3LA (GB)
(74) Representative: D Young & Co LLP
(86) International application number: PCT/GB2018/051238
(87) International publication number: WO 2018/206940

(56) References cited:
- WO-A1-2016/090426
- WO-A1-2016/135959
- WO-A1-2017/001817
- WO-A1-2017/001819
- US-A1- 2015 053 217

## Description

### Field

The present disclosure relates to vapour provision systems such as nicotine delivery systems (e.g. electronic cigarettes and the like).

### Background

Electronic vapour provision systems such as electronic cigarettes (e-cigarettes) generally contain a vapour precursor material, such as a reservoir of a source liquid containing a formulation, typically including nicotine, or a solid material such a tobacco-based product, from which a vapour is generated for inhalation by a user, for example through heat vaporisation. Thus, a vapour provision system will typically comprise a vapour generation chamber containing a vaporiser, e.g. a heating element, arranged to vaporise a portion of precursor material to generate a vapour in the vapour generation chamber. As a user inhales on the device and electrical power is supplied to the vaporiser, air is drawn into the device through inlet holes and into the vapour generation chamber where the air mixes with the vaporised precursor material and forms a condensation aerosol. There is a flow path connecting between the vapour generation chamber and an opening in the mouthpiece so the incoming air drawn through the vapour generation chamber continues along the flow path to the mouthpiece opening, carrying some of the vapour with it, and out through the mouthpiece opening for inhalation by the user.

In some systems the supply of power to the vaporiser is activated manually, for example by a user pressing an activation button when they wish to generate vapour for inhalation. In some other systems the supply of power to the vaporiser is activated automatically in response to user inhalation, for example using a pressure or airflow sensor to detect when a user is inhaling on the device.

The amount of power supplied to the vaporiser of an electronic cigarette when the vaporiser is activated affects the user experience. Broadly speaking, a higher amount of power will generate more vapour so the use can inhale correspondingly more of the components comprising the vapour, such as nicotine and / or flavourants. There can also be other effects, for example different vapour temperatures and condensed vapour particle sizes may be associated with different amounts of power supplied to the vaporiser.

In view of this, some electronic cigarettes allow a user to select different levels of power for the vaporiser during use to tailor they experience. For example, some devices may incorporate two activation buttons with each button being associated with the supply of a different amount of power, thereby in effect providing a button for activating a low power mode and a button for activating a high power mode. Other devices may allow a user to set a desired amount of power to use when the vaporiser is activated, e.g., through a menu system.

While devices that allow a user to select different levels of power for the vaporiser during use thus provide additional desired functionality, the inventors have recognized existing approaches for providing this functionality can in some respects lack flexibility and convenience for users.

Various approaches are described herein which seek to help address or mitigate some of these issues.

### Summary

The invention is defined in independent claims 1 and 15. The dependent claims are preferred embodiments.

According to a first aspect of certain embodiments there is provided a vapour provision system comprising: a first activation sensor; a second activation sensor; user programming circuitry configured, in response to user input, to store a first user-defined power setting for use in association with the first activation sensor and a second user-defined power setting for use in association with the second activation sensor; and power supply control circuitry configured to control a supply of power to a vaporiser to generate vapour from a vapour precursor material for user inhalation, wherein the power supply control circuitry is configured to control the supply of power to the vaporiser in accordance with the first user-defined power setting in response to detecting user activation of the first activation sensor and to control the supply of power to the vaporiser in accordance with the second user-defined power setting in response to detecting user activation of the second activation sensor.

According to another aspect of certain embodiments there is provided a Vapour provision means comprising: first activation means; second activation means; user programming means configured, in response to user input, to store a first user-defined setting for use in association with the first activation means and a second user-defined setting for use in association with the second activation means; and control means configured to control a supply of power to vaporising means to generate vapour from a vapour precursor material for user inhalation, wherein the control means is configured to control the supply of power to the vaporising means in accordance with the first user-defined setting in response to detecting user activation of the first activation means and to control the supply of power to the vaporiser in accordance with the second user-defined setting in response to detecting user activation of the second activation means.

According to another aspect of certain embodiments there is provided a non-therapeutic method of operating a vapour provision system, comprising receiving user input to indicate a first user-defined setting for use in association with a first activation sensor of the vapour provision system and a second user-defined setting for use in association with a second activation sensor of the vapour provision system, and, subsequently, supplying power to a vaporiser to generate vapour from a vapour precursor material for user inhalation in accordance with the first user-defined setting in response to detecting user activation of the first activation sensor and supplying power to the vaporiser to generate vapour from the vapour precursor material for user inhalation in accordance with the second user-defined setting in response to detecting user activation of the second activation sensor.

It will be appreciated that features and aspects of the disclosure described above in relation to the first and other aspects of the disclosure are equally applicable to, and may be combined with, embodiments of the disclosure according to other aspects of the disclosure as appropriate, and not just in the specific combinations described above.

### Brief Description of the Drawings

Embodiments of the invention will now be described, by way of example only, with reference to the accompanying drawings, in which:
Figure 1 represents in highly schematic cross-section a vapour provision system in accordance with certain embodiments of the disclosure;
Figure 2 is a flow diagram representing user programming steps for the vapour provision system of Figure 1;
Figure 3 is a flow diagram representing operating steps for the vapour provision system of

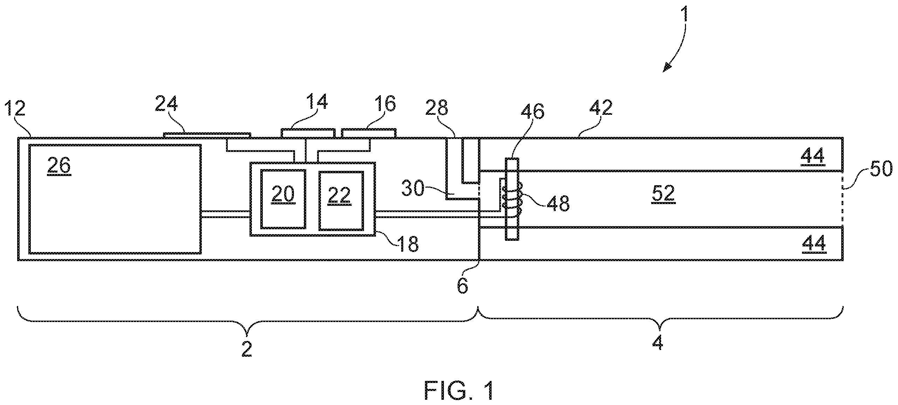

Figure 1 in normal use to generate vapour; and

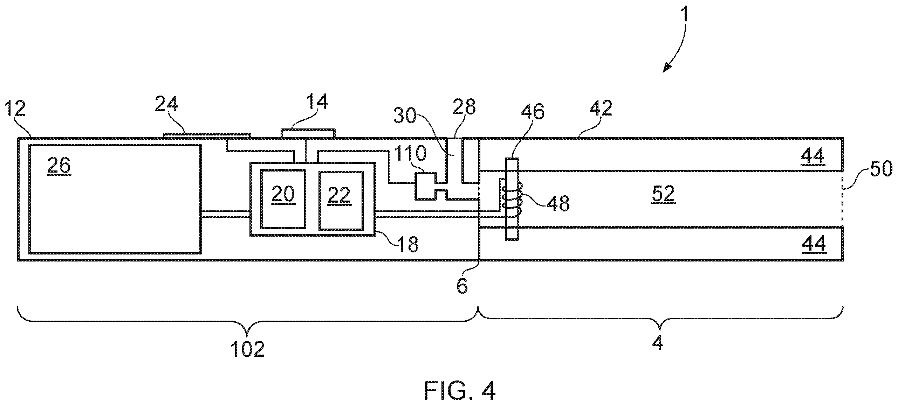

Figure 4 represents in highly schematic cross-section a vapour provision system in accordance with certain other embodiments of the disclosure.

### Detailed Description

Aspects and features of certain examples and embodiments are discussed / described herein. Some aspects and features of certain examples and embodiments may be implemented conventionally and these are not discussed / described in detail in the interests of brevity. It will thus be appreciated that aspects and features of apparatus and methods discussed herein which are not described in detail may be implemented in accordance with any conventional techniques for implementing such aspects and features.

The present disclosure relates to vapour provision systems, which may also be referred to as aerosol provision systems, such as e-cigarettes. Throughout the following description the term "e-cigarette" or "electronic cigarette" may sometimes be used, but it will be appreciated this term may be used interchangeably with vapour provision system / device and electronic vapour provision system / device. Furthermore, and as is common in the technical field, the terms "vapour" and "aerosol", and related terms such as "vaporise", "volatilise" and "aerosolise", may generally be used interchangeably.

Vapour provision systems (e-cigarettes) often, though not always, comprise a modular assembly including both a reusable part and a replaceable (disposable) cartridge part. Often the replaceable cartridge part will comprise the vapour precursor material and the vaporiser and the reusable part will comprise the power supply (e.g. rechargeable battery) and control circuitry. It will be appreciated these different parts may comprise further elements depending on functionality. For example, the reusable device part will often comprise a user interface for receiving user input and displaying operating status characteristics, and the replaceable cartridge part in some cases comprise a temperature sensor for helping to control temperature. Cartridges are electrically and mechanically coupled to a control unit for use, for example using a screw thread or bayonet fixing with appropriately engaging electrical contacts. When the vapour precursor material in a cartridge is exhausted, or the user wishes to switch to a different cartridge having a different vapour precursor material, a cartridge may be removed from the control unit and a replacement cartridge attached in its place. Devices conforming to this type of two-part modular configuration may generally be referred to as two-part devices. It is common for electronic cigarettes to have a generally elongate shape. For the sake of providing a concrete example, certain embodiments of the disclosure described herein will be taken to comprise this kind of generally elongate two-part device employing disposable cartridges. However, it will be appreciated the underlying principles described herein may equally be adopted for different electronic cigarette configurations, for example single part devices or modular devices comprising more than two parts, refillable devices and single-use disposable devices, as well as devices conforming to other overall shapes, for example based on so-called box-mod high performance devices that typically have a more boxy shape. More generally, it will be appreciated certain embodiments of the disclosure are based on electronic cigarettes which are operationally configured to provide functionality in accordance with the principles described herein and the constructional aspects of the electronic cigarettes configured to provide the functionality in accordance with certain embodiments of the disclosure is not of primary significance.

Figure 1 is a cross-sectional view through an example e-cigarette 1 in accordance with certain embodiments of the disclosure. The e-cigarette 1 comprises two main components, namely a reusable part 2 and a replaceable / disposable cartridge part 4. In normal use the reusable part 2 and the cartridge part 4 are releasably coupled together at an interface 6. When the cartridge part is exhausted or the user simply wishes to switch to a different cartridge part, the cartridge part may be removed from the reusable part and a replacement cartridge part attached to the reusable part in its place. The interface 6 provides a structural, electrical and air path connection between the two parts and may be established in accordance with conventional techniques, for example based around a screw thread or bayonet fixing with appropriately arranged electrical contacts and openings for establishing the electrical connection and air path between the two parts as appropriate. The specific manner by which the cartridge part 4 mechanically mounts to the reusable part 2 is not significant to the principles described herein, but for the sake of a concrete example is assumed here to comprise a screw thread fitting (not represented in Figure 1). It will also be appreciated the interface 6 in some implementations may not support an electrical and / or air path connection between the respective parts. For example, in some implementations a vaporiser may be provided in the reusable part rather than in the cartridge part, or the transfer of electrical power from the reusable part to the cartridge part may be wireless (e.g. based on electromagnetic induction), so that an electrical connection between the reusable part and the cartridge part is not needed. Furthermore, in some implementations the airflow through the electronic cigarette might not go through the reusable part so that an air path connection between the reusable part and the cartridge part is not needed.

The cartridge part 4 may in accordance with certain embodiments of the disclosure be broadly conventional. In Figure 1, the cartridge part 4 comprises a cartridge housing 42 formed of a plastics material. The cartridge housing 42 supports other components of the cartridge part and provides the mechanical interface 6 with the reusable part 2. The cartridge housing is generally circularly symmetric about a longitudinal axis along which the cartridge part couples to the reusable part 2. In this example the cartridge part has a length of around 4 cm and a diameter of around 1.5 cm. However, it will be appreciated the specific geometry, and more generally the overall shapes and materials used, may be different in different implementations.

Within the cartridge housing 42 is a reservoir 44 that contains liquid vapour precursor material. The liquid vapour precursor material may be conventional, and may be referred to as e-liquid. The liquid reservoir 44 in this example has an annular shape with an outer wall defined by the cartridge housing 42 and an inner wall that defines an air path 52 through the cartridge part 4. The reservoir 44 is closed at each end with end walls to contain the e-liquid. The reservoir 44 may be formed in accordance with conventional techniques, for example it may comprise a plastics material and be integrally moulded with the cartridge housing 42.

The cartridge part further comprises a wick 46 and a heater (vaporiser) 48 located towards an end of the reservoir 44 opposite to the mouthpiece outlet 50. In this example the wick 46 extends transversely across the cartridge air path 52 with its ends extending into the reservoir 44 of e-liquid through openings in the inner wall of the reservoir 44. The openings in the inner wall of the reservoir are sized to broadly match the dimensions of the wick 46 to provide a reasonable seal against leakage from the liquid reservoir into the cartridge air path without unduly compressing the wick, which may be detrimental to its fluid transfer performance.

The wick 46 and heater 48 are arranged in the cartridge air path 52 such that a region of the cartridge air path 52 around the wick 46 and heater 48 in effect defines a vaporisation region for the cartridge part. E-liquid in the reservoir 44 infiltrates the wick 46 through the ends of the wick extending into the reservoir 44 and is drawn along the wick by surface tension / capillary action (i.e. wicking). The heater 48 in this example comprises an electrically resistive wire coiled around the wick 46. In this example the heater 48 comprises a nickel chrome alloy (Cr20Ni80) wire and the wick 46 comprises a glass fibre bundle, but it will be appreciated the specific vaporiser configuration is not significant to the principles described herein. In use electrical power may be supplied to the heater 48 to vaporise an amount of e-liquid (vapour precursor material) drawn to the vicinity of the heater 48 by the wick 46. Vaporised e-liquid may then become entrained in air drawn along the cartridge air path from the vaporisation region towards the mouthpiece outlet 50 for user inhalation.

As noted above, the rate at which e-liquid is vaporised by the vaporiser (heater) 48 will depend on the amount (level) of power supplied to the heater 48. Thus electrical power can be applied to the heater to selectively generate vapour from the e-liquid in the cartridge part 4, and furthermore, the rate of vapour generation can be changed by changing the amount of power supplied to the heater 48, for example through pulse width and/or frequency modulation techniques.

The reusable part 2 comprises an outer housing 12 having with an opening that defines an air inlet 28 for the e-cigarette, a battery 26 for providing operating power for the electronic cigarette, control circuitry 18 for controlling and monitoring the operation of the electronic cigarette, a first user input button 14, a second user input button 16, and a visual display 24.

The outer housing 12 may be formed, for example, from a plastics or metallic material and in this example has a circular cross section generally conforming to the shape and size of the cartridge part 4 so as to provide a smooth transition between the two parts at the interface 6. In this example the reusable part has a length of around 8 cm so the overall length of the e-cigarette when the cartridge part and reusable part are coupled together is around 12 cm. However, and as already noted, it will be appreciated that the overall shape and scale of an electronic cigarette implementing an embodiment of the disclosure is not significant to the principles described herein.

The air inlet 28 connects to an air path 30 through the reusable part 2. The reusable part air path 30 in turn connects to the cartridge air path 52 across the interface 6 when the reusable part 2 and cartridge part 4 are connected together. Thus, when a user inhales on the mouthpiece opening 50, air is drawn in through the air inlet 28, along the reusable part air path 30, across the interface 6, through the vapour generation region in the vicinity of the atomiser 48 (where vaporised e-liquid becomes entrained in the air flow), along the cartridge air path 52, and out through the mouthpiece opening 50 for user inhalation.

The battery 26 in this example is rechargeable and may be of a conventional type, for example of the kind normally used in electronic cigarettes and other applications requiring provision of relatively high currents over relatively short periods. The battery 26 may be recharged through a charging connector in the reusable part housing 12, for example a USB connector.

The first and second user input buttons 14, 16 in this example are conventional mechanical buttons, for example comprising a sprung mounted component which may be pressed by a user to establish an electrical contact. In this regard, the input buttons may be considered input devices for detecting user input and the specific manner in which the buttons are implemented is not significant. For example, other forms of mechanical button(s) or touch-sensitive button(s) (e.g. based on capacitive or optical sensing techniques) may be used in other implementations. The specific manner in which the buttons are implemented may in particular be selected having regard to a desired aesthetic appearance. For example, in some cases the two buttons 14, 16 may be structurally independent of one another, whereas in other cases the two buttons 14, 16 may be to some extent integrated, for example with a single toggle element used to selectively activate one or other or both of the respective buttons.

The display 24 is provided to give a user with a visual indication of various characteristics associated with the electronic cigarette, for example current power setting information, remaining battery power, and so forth. The display may be implemented in various ways. In this example the display 24 comprises a conventional pixilated LCD screen that may be driven to display the desired information in accordance with conventional techniques. In other implementations the display may comprise one or more discrete indicators, for example LEDs, that are arranged to display the desired information, for example through particular colours and / or flash sequences. More generally, the manner in which the display is provided and information is displayed to a user using the display is not significant to the principles described herein. For example some embodiment may not include a visual display and may include other means for providing a user with information relating to operating characteristics of the electronic cigarette, for example using audio signalling, or may not include any means for providing a user with information relating to operating characteristics of the electronic cigarette.

The control circuitry 18 is suitably configured / programmed to control the operation of the electronic cigarette to provide functionality in accordance with embodiments of the disclosure as described further herein, as well as for providing conventional operating functions of the electronic cigarette in line with the established techniques for controlling such devices. The control circuitry (processor circuitry) 18 may be considered to logically comprise various subunits / circuitry elements associated with different aspects of the electronic cigarette's operation. In this example the control circuitry 18 comprises power supply control circuitry 22 for controlling the supply of power from the battery 26 to the vaporiser 48 in response to user input, user programming circuitry 20 for establishing configuration settings (e.g. user-defined power settings) in response to user input, as well as other functional units / circuitry associated functionality in accordance with the principles described herein and conventional operating aspects of electronic cigarettes, such as display driving circuitry and user input detection circuitry. It will be appreciated the functionality of the control circuitry 18 can be provided in various different ways, for example using one or more suitably programmed programmable computer(s) and / or one or more suitably configured application-specific integrated circuit(s) / circuitry / chip(s) / chipset(s) configured to provide the desired functionality.

Thus the vapour provision system 1 comprises a first activation sensor for detecting user activation (i.e. pressing) of the first button 14 and a second activation sensor for detecting user activation (i.e. pressing) of the second button 16. As discussed further herein, the control circuitry 18, and more particularly the logical component of the control circuitry comprising the power supply control circuitry 22, is configured to control a supply of power from the battery 26 to the heater / vaporiser 48 to generate vapour from a portion of the e-liquid in the cartridge part 4 for user inhalation via the mouthpiece outlet 50 in response to user activation of one or other (or both) of the activation sensors. Each activation sensor is associated with an amount of power to supply to the vaporiser that is user-configured. That is to say, a user may program the control circuitry 18, and more particularly the logical component of the control circuitry comprising the user programming circuitry 22, to associate each activation sensor with a different user-defined power level setting. Thus, for example, a user may program the vapour provision system 1 so as to supply a relatively low amount of power to the vaporiser 48 when the user presses the first button 14 and to supply a relatively high amount of power to the vaporiser 48 when the user presses the second button 16. In some example implementations, the vapour provision system 1 may further be configured to supply a third amount of power to the vaporiser 48 when the user presses the first button 14 and the second button simultaneously. In some cases the third amount of power may also be user-defined and configurable by the user programming circuitry, thereby providing the user with the flexibility to define three separate performance / vapour generation levels that can be directly accessed by simply pressing the appropriate button(s). In some other cases the third amount of power may be predefined, for example it may correspond with the maximum power that can be provided by the battery, thereby providing the user with the flexibility to define two separate power levels that can be directly accessed by simply pressing the appropriate button as well as the ability to supply maximum power to the vaporiser by pressing both buttons together.

Thus, in accordance with the principles described herein, a user is provided with the ability to activate multiple different user-defined power levels. The inventors have recognised this provides improved flexibility and convenience over existing schemes which rely on supplying fixed amounts of power or requiring a user to enter a programming menu to change the amount of power. For example, a user may wish to use a relatively high level of power for vapour generation at the beginning of a use session, but to use a lower level of power for vapour generation towards the end of a use session. This may be achieved in accordance with certain embodiments of the disclosure described herein by simply associating each of the desired power levels with one of the buttons, and then using the relevant button to activate vapour generation accordingly.

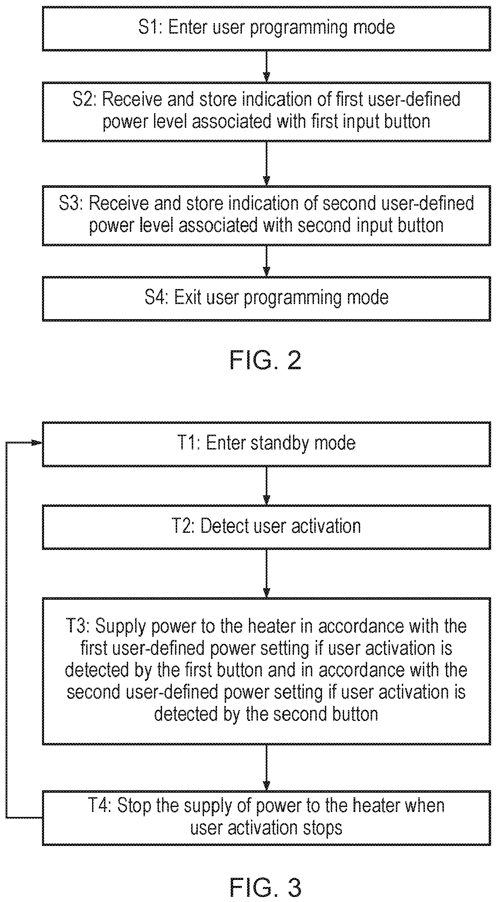

Figure 2 is a flow diagram schematically representing a method of end-user programming of a vapour provision system to associate the desired user-defined power level settings with the respective activation sensors in accordance with certain embodiments of the disclosure. In this particular example it is assumed the first button 14 and the second button 16 are used to provide user input for programming the vapour provision system. However, it will be appreciated other example implementations may have one or more additional user input devices, for example one or more additional button(s), for user input during programming. In yet other examples embodiments, a vapour provision system may instead, or additionally, be configured to connect (wired or wirelessly) to a separate device, for example a computer, such as a smartphone or tablet, running an application to allow the user to program the vapour provision system, and in particular to configure the user-defined power level settings to associate with the different activation sensors. More generally any approach may be provided to allow a user to established settings for the different activation means provided by a device to correspond with the user's desired levels of vapour generation for the different activation means

In step S1 of Figure 2, the vapour provision system is caused to enter a user programming mode. This may be achieved, for example, by a user pressing the first and / or second buttons in a predefined sequence, e.g. pressing the first button six times within three seconds, or alternately pressing each button three times. It will, of course, be appreciated the specific manner in which the vapour provision system is triggered to enter the user programming mode is not significant to the principles described herein. In general, a user may enter the user programming mode whenever they want to change the power settings currently associated with the different activation sensors (which may be factory defaults if they have not previously been set). When the vapour provision system enters the user programming mode the display may be configured to provide the user with an indication of this, as well as other information, such as current configuration settings.

In step S2, when in the user programming mode, the user provides user input to indicate a desired power level setting to be associated with the first input button. There are many ways this can be done. For example, in some cases a user may define the desired power level setting in terms of indicating a specific number of watts, for example by using the first and second buttons to respectively increment and decrement a present power level setting indicated by the visual display 24, for example in steps of 0.1 watts, or whichever resolution is provided in a given implementation. In some cases the desired power level settings may be associated with arbitrary units, for example a user may be able to select from 10 different settings between zero power and maximum power. Again, when in the user programming mode, a user may use the first and second input buttons to adjust the current power level setting on such a scale. The user may confirm the desired power level setting for association with the first input button, for example by pressing one or other of the buttons in a predefined sequence, e.g. three quick presses of one or other button, or simultaneous press of both buttons, which is taken to in effect correspond to the user indicating "ok" or "enter".

In step S3, when in the user programming mode and after having received and stored the indication of the first user-defined power level associated with the first input button, the user programming circuitry may, in response to user input, receive and store an indication of a second user-defined power level associated with the second input button, which may be done in the same way as for step S2.

In step S4, with the first and second user-defined power levels having been set, the user may exit the programming mode. Again, this may be achieved in any of a number of different ways, for example by pressing one or other of the user input buttons in a predefined sequence taken to correspond with an instruction to exit the user programming mode.

Thus, Figure 2 shows one way in which a user may establish their desired power settings for association with the first and second input buttons. It will be appreciated this provides merely one of many different ways this can be achieved, for example having regard to the established practices for user programming of electronic devices, such as electronic cigarettes. It will also be appreciated the order in which the power levels are set is of course not significant. Furthermore, it will be appreciated the user programming mode may also be used to set any other configurable aspects of the vapour provision system that may be available for a given implementation.

Having programmed the vapour provision system, and in particular having configured a first user-defined power setting for use in association with the first button 14 and a second user-defined power setting for use in association with the second button 16, and having exited the programming mode, the vapour provision system is ready for normal use (i.e. selective vapour generation).

Figure 3 is a flow diagram schematically representing a non-therapeutic method of using the vapour provision system of Figure 1 to selectively generate vapour in accordance with certain embodiments of the disclosure.

In step T1 the vapour provision system 1 enters a standby state. As is common for electronic cigarettes, the vapour provision system 1 supports three basic operating states, namely an "off" state, an "on" state, and a "standby" state. In the off state, the electronic cigarette is unable to generate vapour (i.e. the power supply control circuitry is prevented from supplying power to the vaporiser / heater in the off state). The electronic cigarette may, for example, be placed in the off state between use sessions, for example when the electronic cigarette might be set aside or placed in a user's pocket or bag. In the on (or active) state, the electronic cigarette is actively generating vapour (i.e. the power supply control circuitry is supplying power to the vaporiser / heater). The electronic cigarette will thus typically be in the on state when a user is in the process of inhaling vapour from the electronic cigarette. In the standby state the electronic cigarette is ready to generate vapour (i.e. ready to apply power to the vaporiser) in response to user input, but is not currently doing so. The electronic cigarette will thus typically be in the standby state when a user initially exits the off state to begin a session of use, or between inhalations during an ongoing session of use. For the sake of a concrete example, it is assumed that in step T1 represented in Figure 3 the electronic cigarette enters the standby state by virtue of the user bringing the device out of the off state to begin a session of use. However, the processing represented in Figure 3 is the same for when the electronic cigarette enters the standby state from the on state because a user has finished inhaling on the electronic cigarette. The manner in which the electronic cigarette is caused to switch from the off state to the standby state will be a matter of implementation and is not significant here. For example, to transition from the off state to the standby state the user may be required to press one of the input buttons in a particular sequence, for example multiple presses within a predetermined time, or to press both buttons together.

In step T2, the power supply control circuitry detects user activation corresponding to a user pressing one or other of the first and second input buttons 14, 16. In this regard, the user will press whichever button corresponds with the power level setting they would currently like to use based on their previously-configured user-defined power level settings.

In step T3, the power supply control circuitry supplies power to the vaporiser in accordance with the relevant power level setting. That is to say, if in step T2 the detected user activation corresponds with the user pressing the first user input button 14, the power supply control circuitry supplies power to the vaporiser in accordance with the user-defined power level setting previously associated with the first button during the programming process represented in Figure 2. If, on the other hand, in step T2 the detected user activation corresponds with the user pressing the second user input button 16, the power supply control circuitry supplies power to the vaporiser in accordance with the user-defined power level setting previously associated with the second button during the programming process represented in Figure 2.

The manner in which the power supply is controlled to correspond with the relevant power level setting will depend on the implementation at hand, and may be generally based on conventional techniques. Typically, it may be expected different power level settings will be associated with different fill-factors / duty cycles in a pulse- or frequency-width modulation scheme for supplying power to the vaporiser (in that sense it will be appreciated the supply of power to the vaporiser during vapour generation will typically change, e.g. by pulsing a drive current on and off, on a timescale that is typically faster than a cooling / heating time for the vaporiser and it is the average power level that is most relevant for practical purposes). However, in principle the power supply control circuitry may equally be configured to supply power at a constant voltage that is different for different power level settings. In general, the range of different power level settings that are available for the user to select from during programming and the manner in which the power supply control circuitry is configured to supply electric current to the vaporiser in correspondence with the different power level settings is not significant to the principles described herein.

Furthermore, it will be appreciated the user setting need not specify a specific amount of power to deliver to the vaporiser, but may be based on a different parameterisation. For example, in some implementations an electronic cigarette may allow a user to select a desired temperature setting for vapour generation and the power supply circuitry for the electronic cigarette will be configured to supply power at a level which is appropriate for maintaining the desired temperature setting (i.e. the specific power supplied at different points during a puff will vary according to what is needed to maintain a temperature corresponding to the desired temperature level setting. In such cases a user may associate a desired a user-defined temperature level setting with different activation sensors as opposed to a user-defined power level setting. However, and as is common in the field, the phrase "power level setting" may nonetheless typically be used even when the setting does not in itself relates directly to an amount of power delivered to the heater. Rather, the phrase power level setting may be used more generally to refer to a setting that impacts a userperceived level of vapour generation.

In Step T4 the power supply control circuitry detects the end of the user activation initiated in step T2, which in this example corresponds with the user releasing the button they started pressing in step T2. In response to this the power supply control circuitry operates to stop the supply of power to the vaporiser, thereby ending the current inhalation / puff event. In some implementations the power supply control circuitry may also be configured to stop supplying power to the vaporiser if certain other conditions are met. For example, the supply of power may be stopped if a predefined maximum time for vapour generation for a single activation event is reached or a fault condition is detected (e.g. overheating).

Thus, in accordance with the processing represented Figure 3, a user may be provided with vapour generated in accordance with their desired power setting for inhalation. Significantly, the user is able to select from multiple, in this case two but in other examples more, user-defined power settings on a puff-by-puff basis (and in principle within a single puff by switching buttons) without needing to reconfigure or reprogram the electronic cigarette to change between the two user-defined power settings. This approach increases the flexibility and convenience of operation for users.

It will be appreciated the vapour provision system and processing discussed above in relation to Figures 1 to 3 may be modified in various ways for different implementations.

For example, in this example implementation it is assumed power is supplied to the vaporiser whenever a user is pressing one of the user input buttons 14, 16. However, in other implementations the electronic cigarette may further include an inhalation sensor, for example a pressure sensor, configured to detect when a user is actively inhaling on the electronic cigarette. In such cases the power supply control circuitry may be configured to only supply power to the vaporiser in response to user activation of one or other of the user input buttons when the inhalation sensor detects the user is actively inhaling on the electronic cigarette. In such cases the user may need to maintain pressure on the relevant input button to maintain the generation of vapour during their inhalation, or they may simply press the relevant button at the beginning, or before, inhaling to indicate the desired power level, which may then be supplied for so long as the user continues inhaling, regardless of whether the user continues pressing the relevant button during the inhalation event. In that sense, activation of one of the activation sensors associated with the different pre-stored user-defined power level settings may be considered to in effect correspond with detecting a user is pressing the relevant button in association with (i.e. at or around the same time as) inhaling on the electronic cigarette.

Figure 4 is a cross-sectional view through an example vapour provision system 101 that represents a variation of the vapour provision system 1 represented in Figure 1 in accordance with certain other embodiments of the disclosure. Elements of the vapour provision system 101 represented in Figure 4 which are functionally and / or structurally similar to, and will be understood from, corresponding elements of the vapour provision system 1 represented in Figure 1 are identified with corresponding reference numerals and are not discussed again in detail in the interests of brevity. The electronic cigarette 101 represented in Figure 4 differs from the electronic cigarette 1 represented in Figure 1 by the manner in which its activation sensors detect user activation events. In particular, whereas the activation sensors for the electronic cigarette 1 represented in Figure 1 are based on two user input buttons, the activation sensors for the electronic cigarette 101 represented in Figure 4 are based on a combination of one user input button 14 and an inhalation sensor 110.

Thus the electronic cigarette 101 in Figure 4 again comprises two main components, namely a reusable part 102 and a replaceable / disposable cartridge part 4. The cartridge part for the electronic cigarette 101 represented in Figure 4 may be identical to the cartridge part for the electronic cigarette 1 represented in Figure 1. However, and as noted above, the reusable part 102 of the electronic cigarette represented in Figure 4 differs from the reusable part 2 of the electronic cigarette represented in Figure 1 in only having one user input button 14 and including an inhalation sensor 110. The inhalation sensor 110 is provided to allow the power supply control circuitry 22 to determine when a user inhales on the mouthpiece outlet 50 of the electronic cigarette 101. The inhalation sensor 110 may be based on any conventional inhalation sensing technique and will typically comprise a pressure sensor in a chamber in fluid communication with the air path 30 in the reusable part 102. When a user inhales on the electronic cigarette the corresponding pressure drop within the air path 30 is detected by the power supply control circuitry based on measurements from the inhalation sensor 110.

In a manner similar to that described above with reference to Figure 2, a user of the electronic cigarette 101 represented in Figure 4 may program first and second user-defined power levels to be associated with first and second activation sensors in broadly the same way as for the electronic cigarette 1 represented in Figure 1. However, what is different between these two electronic cigarettes is the manner in which a user activates a desired one of the two user-defined power level settings. In the example electronic cigarette 1 represented in Figure 1 the first activation sensor is based on detecting a user pressing on the first user input button 14 and the second activation sensor is based on detecting a user pressing on the second of user input button. However, in the example electronic cigarette 101 represented in Figure 4, the first activation sensor is based on detecting a user inhaling on the electronic cigarette using the inhalation sensor 110 and the second activation sensor is based on detecting a user pressing on the user input button 14 in conjunction with (i.e. at or around the same time as) inhaling on the electronic cigarette.

Thus, in a user programming step for the electronic cigarette 101 represented in Figure 4, a first user-defined power setting level may be associated with a user inhaling on the device without pressing the button 14 while a second user-defined power setting level may be associated with a user inhaling on the device at the same time as pressing the button 14.

During use the electronic cigarette 101 represented in Figure 4 may operate generally in accordance with the procedure represented in Figure 3, except that in step T3, the power supply control circuitry 22 is configured to supply power to the heater in accordance with the first user-defined power level setting when the user inhales on the electronic cigarette without pressing the button 14, and to supply power to the heater in accordance with the second user-defined power level setting when the user inhales on the electronic cigarette in conjunction with pressing the button 14. In that sense, a user may find it convenient to set the first power level to correspond to the lower of their two desired power level settings and to set the second power level to correspond to the higher of their two desired power level settings. With this approach, the use of the button 14 in conjunction with inhalation on the electronic cigarette may be considered to in effect provide a user-defined power boost in that pressing the button will increase the amount of power supplied to the vaporiser when the user inhales on the electronic cigarette from the lower of the user's desired power level setting to the higher of the user's desired power level settings. Depending on the implementation at hand, in some cases the user may be required to maintain pressure on the input button to maintain the generation of vapour at the second power level setting during their inhalation, and if the user releases the button but continues inhaling, the power supplied to the vaporiser will change to the first power level setting. Alternatively, the system may be configured to operate such that pressing the the input button at or just before the beginning of inhalation causes the second power level setting to be used throughout the inhalation. Similarly, it would be a matter for implementation as to whether the power supply to the vaporiser is changed if the input button is pressed part way through an ongoing inhalation.

It will be appreciated there are various other ways in which an electronic cigarette configured to operate in accordance with the principles described herein may be associated with approaches for user activation of different activation sensors. For example, in another implementation an electronic cigarette may comprise an inhalation sensor and a single button similar to that shown in Figure 4, and may be configured such that its first activation sensor is triggered by a user pressing the button with a single press and its second activation sensor is triggered by a user pressing the button with multiple presses in a predetermined time, for example two presses within one second. A user may thus indicate the user-defined power setting they wish to use based on whether they press the button once or twice, and then proceed to inhale on the device to trigger the supply of power to the vaporiser in accordance with their selected user-defined power level setting. More generally, the different activation sensors may be associated with detecting that a user presses the button in one of a number of different predefined pressing sequences.

While the above-described embodiments have in some respects focussed on some specific example vapour provision systems, it will be appreciated the same principles can be applied for vapour provision systems using other technologies. That is to say, the specific manner in which various aspects of the vapour provision system function are not directly relevant to the principles underlying the examples described herein.

For example, whereas the above-described embodiments have primarily focused on devices having an electrical heater based vaporiser for heating a liquid vapour precursor material, the same principles may be adopted in accordance with vaporisers based on other technologies, for example piezoelectric vibrator based vaporisers or optical heating vaporisers, and also devices based on other aerosol precursor materials, for example solid materials, such as plant derived materials, such as tobacco derivative materials, or other forms of vapour precursor materials, such as gel, paste or foam based vapour precursor materials.

It will also be appreciated that while the above-described examples have focused on implementations comprising two user-defined power settings with corresponding user inputs, in other implementations, the same principles may be applied in respect of vapour provision systems supporting more than two user-defined power settings and corresponding user inputs. For example, a vapour provision system in accordance with some implementations may support three, four or more user-defined power settings with a corresponding number of user inputs / activation sensors.

Furthermore, while the above-described embodiments have focused on approaches in which a user may associate a given power level setting with activation of a given user activation sensor, in other example implementations, the vapour provision system may also allow a user to configure other aspects of power deliver for association with each of a number of different user activation sensors. For example, rather than simply define a fixed power level setting to associate with a particular button (or other activation sensor), a vapour provision system according to certain embodiments may allow a user to define a time-varying profile of power supply throughout a puff. For example, puffs (inhalation events) may be notionally divided into a series of segments, for example ten half-second segments (giving a maximum puff duration of five seconds), and a user may program a desired power level setting for each of the segments in a puff using a programming mode of the kind discussed above. A user may thus program different time-varying power supply profiles for different activation sensors, so that, in normal use, the user may activate one button to deliver a first power supply profile during a puff, and another button to deliver a second, different, power supply profile during a puff. For example, one puff profile may comprise a high-power start (e.g. maximum power for the first two seconds) with a lower-power tail (e.g. two-thirds maximum power for next two seconds, and one half maximum power for the final second), while another puff profile may comprise a steady power delivery (e.g. half power through a complete puff). A user may thus, for example, use the first power profile by activating the first button to gain an initial impact at the beginning of a use session, and proceed to use the more steady power delivery profile for the remaining puffs in the use session (by activating the second button).

It will be appreciated there are various ways in which the user may define the various puff profiles to associate with the different activation sensors. For example, a user may be able to configure the time resolution for the different segments of the power delivery profile and / or a maximum duration of a puff profile (i.e. number of different segments), or these may be fixed. Furthermore, rather than have a user input specific power levels / power level settings for each segment of a puff, in some examples including an inhalation sensor, the vapour provision system may be configured to learn a desired puff profile based on the profile of a user's inhalation during a programming session. For example, the vapour provision system might comprise an in-built puff response mode whereby the amount of power supplied at different points during a puff is responsive to the current strength of a user's inhalation (e.g. based on pressure measurements). When in the programming mode, this in-built puff response mode may be used to map a user's inhalation profile during programming to a corresponding a power supply profile, and associate this with one of the activation sensors. Subsequently, when in normal use, when a user activates the relevant activation sensor, the corresponding power supply profile may be provided to the vaporiser. This therefore provides a mechanism for providing a user with an easy mechanism for establishing a desired power delivery profile to associate with an individual button (or other activation means). Furthermore, it can allow a user to provide a desired variation in power delivery throughout a puff, without the vapour provision system needing to continuously measure and moderate power delivery based on current airflow measurements.

Thus there has been described a vapour provision system comprising: a first activation sensor for detecting a first user activation action; a second activation sensor for detecting a second user activation action; user programming circuitry configured, in response to user input, to store a first user-defined setting for use in association with the first activation sensor and a second user-defined setting for use in association with the second activation sensor; and power supply control circuitry configured to control a supply of power to a vaporiser to generate vapour from a vapour precursor material for user inhalation, wherein the power supply control circuitry is configured to control the supply of power to the vaporiser in accordance with the first user-defined setting in response to detecting user activation of the first activation sensor and to control the supply of power to the vaporiser in accordance with the second user-defined setting in response to detecting user activation of the second activation sensor. The first and / or second user activation actions which are detected by the respective sensors may, for example, comprise a user pressing a specific button associated with the activation sensor or a common button in a predefined sequence pattern, or a user inhaling on the system.

In order to address various issues and advance the art, this disclosure shows by way of illustration various embodiments in which the claimed invention(s) may be practiced. The advantages and features of the disclosure are of a representative sample of embodiments only, and are not exhaustive and / or exclusive. They are presented only to assist in understanding and to teach the claimed invention(s). It is to be understood that advantages, embodiments, examples, functions, features, structures, and / or other aspects of the disclosure are not to be considered limitations on the disclosure as defined by the claims or limitations on equivalents to the claims, and that other embodiments may be utilised and modifications may be made without departing from the scope of the claims. Various embodiments may suitably comprise, consist of, or consist essentially of, various combinations of the disclosed elements, components, features, parts, steps, means, etc. other than those specifically described herein, and it will thus be appreciated that features of the dependent claims may be combined with features of the independent claims in combinations other than those explicitly set out in the claims. The disclosure may include other inventions not presently claimed, but which may be claimed in future.

## Claims

1. A vapour provision system (1) comprising:
a first activation sensor (14);
a second activation sensor (16);
user programming circuitry (20) configured, in response to user input, to store a first user-defined power setting for use in association with the first activation sensor and a second user-defined power setting for use in association with the second activation sensor; and
power supply control circuitry (22) configured to control a supply of power to a vaporiser (48) to generate vapour from a vapour precursor material for user inhalation, wherein the power supply control circuitry is configured to control the supply of power to the vaporiser in accordance with the first user-defined power setting in response to detecting user activation of the first activation sensor and to control the supply of power to the vaporiser in accordance with the second user-defined power setting in response to detecting user activation of the second activation sensor.

2. The vapour provision system of claim 1, wherein the first activation sensor is configured to detect user activation in response to a user pressing a first button of the vapour provision system and the second activation sensor is configured to detect user activation in response to a user pressing a second button of the vapour provision system.

3. The vapour provision system of claim 1, wherein the first activation sensor is configured to detect user activation in response to a user pressing a button of the vapour provision system in accordance with a first predefined pressing sequence and the second activation sensor is configured to detect user activation in response to a user pressing the button in accordance with a second predefined pressing sequence.

4. The vapour provision system of claim 3, wherein at least one of the first pressing sequence and the second pressing sequence corresponds with the user pressing the button a predefined number of times within a predefined time.

5. The vapour provision system of claim 1, wherein the first activation sensor is configured to detect user activation in response to a user inhaling on the vapour provision system.

6. The vapour provision system of any of claims 1 to 5, wherein the power supply control circuitry is configured to control the supply of power to the vaporiser in accordance with the second user-defined power setting only when user activation of the second activation sensor is detected in conjunction with user activation of the first activation sensor.

7. The vapour provision system of any of claims 1 to 6, wherein the power supply control circuitry is configured to control the supply of power to the vaporiser in accordance with a third power setting in response to detecting simultaneous user activation of the first activation sensor and the second activation sensor.

8. The vapour provision system of claim 7, wherein the third power setting is a user-defined power setting and the user programming circuitry is configured, in response to user input, to receive and store the third user-defined power setting.

9. The vapour provision system of any of claims 1 to 8, wherein the user programming circuitry is configured to receive user input through the same user input mechanism as the first activation sensor and / or the second activation sensor.

10. The vapour provision system of any of claims 1 to 9, further comprising a display (24) for providing an indication of the first user-defined power setting and / or an indication of the second user-defined power setting.

11. The vapour provision system of any of claims 1 to 10, further comprising a further activation sensor, and wherein the user programming circuitry is further configured, in response to user input, to receive and store a further user-defined power setting for use in association with the further activation sensor, and the power supply control circuitry is configured to control the supply of power to the vaporiser in accordance with the further user-defined power setting in response to detecting user activation of the further activation sensor.

12. The vapour provision system of any of claims 1 to 11, wherein the vapour provision system further comprises the vaporiser.

13. The vapour provision system of any of claims 1 to 12, wherein the vapour provision system further comprises the vapour precursor material.

14. The vapour provision system of any of claims 1 to 13, wherein the vapour provision system is formed from a reusable part (2) comprising the first activation sensor; second activation sensor; user programming circuitry; power supply control circuitry and a power supply, and a cartridge part (4) comprising the vapour precursor material, wherein the cartridge part is releasably couplable to the reusable part for use.

15. A non-therapeutic method of operating a vapour provision system (1), comprising receiving user input to indicate a first user-defined power setting for use in association with a first activation sensor (14) of the vapour provision system and a second user-defined power setting for use in association with a second activation sensor (16) of the vapour provision system, and, subsequently, supplying power to a vaporiser (48) to generate vapour from a vapour precursor material for user inhalation in accordance with the first user-defined power setting in response to detecting user activation of the first activation sensor and supplying power to the vaporiser to generate vapour from the vapour precursor material for user inhalation in accordance with the second user-defined power setting in response to detecting user activation of the second activation sensor.

## Patentansprüche

1. Dampfbereitstellungssystem (1), das Folgendes umfasst:
einen ersten Aktivierungssensor (14);
einen zweiten Aktivierungssensor (16);
eine Anwenderprogrammierschaltungsanordnung (20), die konfiguriert ist, in Reaktion auf eine Anwendereingabe eine erste anwenderdefinierte Leistungseinstellung zur Verwendung in Verbindung mit dem ersten Aktivierungssensor und eine zweite anwenderdefinierte Leistungseinstellung zur Verwendung in Verbindung mit dem zweiten Aktivierungssensor zu speichern; und
eine Leistungsversorgungs-Steuerschaltungsanordnung (22), die konfiguriert ist, eine Leistungsversorgung zu einem Verdampfer (48) zu steuern, um Dampf zur Anwenderinhalation aus einem Dampfvorläufermaterial zu erzeugen, wobei die Leistungsversorgungs-Steuerschaltungsanordnung konfiguriert ist, in Reaktion auf ein Detektieren einer Anwenderaktivierung des ersten Aktivierungssensors die Leistungsversorgung zum Verdampfer in Übereinstimmung mit der ersten anwenderdefinierten Leistungseinstellung zu steuern und in Reaktion auf ein Detektieren einer Anwenderaktivierung des zweiten Aktivierungssensors die Leistungsversorgung zum Verdampfer in Übereinstimmung mit der zweiten anwenderdefinierten Leistungseinstellung zu steuern.

2. Dampfbereitstellungssystem nach Anspruch 1, wobei der erste Aktivierungssensor konfiguriert ist, eine Anwenderaktivierung in Reaktion darauf zu detektieren, dass ein Anwender eine erste Taste des Dampfbereitstellungssystems drückt, und der zweite Aktivierungssensor konfiguriert ist, eine Anwenderaktivierung in Reaktion darauf zu detektieren, dass ein Anwender eine zweite Taste des Dampfbereitstellungssystems drückt.

3. Dampfbereitstellungssystem nach Anspruch 1, wobei der erste Aktivierungssensor konfiguriert ist, eine Anwenderaktivierung in Reaktion darauf zu detektieren, dass ein Anwender eine Taste des Dampfbereitstellungssystems in Übereinstimmung mit einer ersten im Voraus definierten Drückabfolge drückt, und der zweite Aktivierungssensor konfiguriert ist, eine Anwenderaktivierung in Reaktion darauf zu detektieren, dass ein Anwender die Taste in Übereinstimmung mit einer zweiten im Voraus definierten Drückabfolge drückt.

4. Dampfbereitstellungssystem nach Anspruch 3, wobei die erste Drückabfolge und/oder die zweite Drückabfolge damit übereinstimmt, dass der Anwender die Taste eine im Voraus definierte Anzahl von Malen in einer im Voraus definierten Zeit drückt.

5. Dampfbereitstellungssystem nach Anspruch 1, wobei der erste Aktivierungssensor konfiguriert ist, eine Anwenderaktivierung in Reaktion darauf zu detektieren, dass ein Anwender am Dampfbereitstellungssystem inhaliert.

6. Dampfbereitstellungssystem nach einem der Ansprüche 1 bis 5, wobei die Leistungsversorgungs-Steuerschaltungsanordnung konfiguriert ist, die Leistungsversorgung zum Verdampfer lediglich dann in Übereinstimmung mit der zweiten anwenderdefinierten Leistungseinstellung zu steuern, wenn eine Anwenderaktivierung des zweiten Aktivierungssensors in Verbindung mit einer Anwenderaktivierung des ersten Aktivierungssensors detektiert wird.

7. Dampfbereitstellungssystem nach einem der Ansprüche 1 bis 6, wobei die Leistungsversorgungs-Steuerschaltungsanordnung konfiguriert ist, in Reaktion auf ein Detektieren einer gleichzeitigen Anwenderaktivierung des ersten Aktivierungssensors und des zweiten Aktivierungssensors die Leistungsversorgung zum Verdampfer in Übereinstimmung mit einer dritten Leistungseinstellung zu steuern.

8. Dampfbereitstellungssystem nach Anspruch 7, wobei die dritte Leistungseinstellung eine anwenderdefinierte Leistungseinstellung ist und die Anwenderprogrammierschaltungsanordnung konfiguriert ist, in Reaktion auf eine Anwendereingabe die dritte anwenderdefinierte Leistungseinstellung zu empfangen und zu speichern.

9. Dampfbereitstellungssystem nach einem der Ansprüche 1 bis 8, wobei die Anwenderprogrammierschaltungsanordnung konfiguriert ist, eine Anwendereingabe durch denselben Anwendereingabemechanismus wie der erste Aktivierungssensor und/oder der zweite Aktivierungssensor zu empfangen.

10. Dampfbereitstellungssystem nach einem der Ansprüche 1 bis 9, das ferner eine Anzeigevorrichtung (24) zum Bereitstellen einer Anzeige der ersten anwenderdefinierten Leistungseinstellung und/oder einer Anzeige der zweiten anwenderdefinierten Leistungseinstellung umfasst.

11. Dampfbereitstellungssystem nach einem der Ansprüche 1 bis 10, das ferner einen weiteren Aktivierungssensor umfasst, wobei die Anwenderprogrammierschaltungsanordnung ferner konfiguriert ist, in Reaktion auf eine Anwendereingabe eine weitere anwenderdefinierte Leistungseinstellung zur Verwendung in Verbindung mit dem weiteren Aktivierungssensor zu empfangen und zu speichern, und die Leistungsversorgungs-Steuerschaltungsanordnung konfiguriert ist, in Reaktion auf ein Detektieren einer Anwenderaktivierung des weiteren Aktivierungssensors die Leistungsversorgung zum Verdampfer in Übereinstimmung mit der weiteren anwenderdefinierten Leistungseinstellung zu steuern.

12. Dampfbereitstellungssystem nach einem der Ansprüche 1 bis 11, wobei das Dampfbereitstellungssystem ferner den Verdampfer umfasst.

13. Dampfbereitstellungssystem nach einem der Ansprüche 1 bis 12, wobei das Dampfbereitstellungssystem ferner das Dampfvorläufermaterial umfasst.

14. Dampfbereitstellungssystem nach einem der Ansprüche 1 bis 13, wobei das Dampfbereitstellungssystem aus einem wiederverwendbaren Teil (2), der den ersten Aktivierungssensor, den zweiten Aktivierungssensor, eine Anwenderprogrammierschaltungsanordnung, eine Leistungsversorgungs-Steuerschaltungsanordnung und eine Leistungsversorgung umfasst, und einem Kartuschenabschnitt (4), der das Dampfvorläufermaterial umfasst, gebildet ist, wobei der Kartuschenabschnitt zur Verwendung an den wiederverwendbaren Teil lösbar koppelbar ist.

15. Nichttherapeutisches Verfahren zum Betreiben eines Dampfbereitstellungssystems (1), das ein Empfangen einer Anwendereingabe, um eine erste anwenderdefinierte Leistungseinstellung zur Verwendung in Verbindung mit einem ersten Aktivierungssensor (14) des Dampfbereitstellungssystems und eine zweite anwenderdefinierte Leistungseinstellung zur Verwendung in Verbindung mit einem zweiten Aktivierungssensor (16) des Dampfbereitstellungssystems anzugeben, und anschließend ein Zuführen von Leistung zu einem Verdampfer (48), um in Reaktion auf ein Detektieren einer Anwenderaktivierung des ersten Aktivierungssensors aus einem Dampfvorläufermaterial Dampf zur Anwenderinhalation in Übereinstimmung mit der ersten anwenderdefinierten Leistungseinstellung zu erzeugen, und ein Zuführen von Leistung zum Verdampfer, um in Reaktion auf ein Detektieren einer Anwenderaktivierung des zweiten Aktivierungssensors aus dem Dampfvorläufermaterial Dampf zur Anwenderinhalation in Übereinstimmung mit der zweiten anwenderdefinierten Leistungseinstellung zu erzeugen, umfasst.

## Revendications

1. Système de délivrance de vapeur (1) comprenant :
un premier capteur d'activation (14) ;
un deuxième capteur d'activation (16) ;
un circuit de programmation utilisateur (20) configuré, en réponse à une entrée utilisateur, pour stocker un premier réglage de puissance défini par l'utilisateur à utiliser en association avec le premier capteur d'activation et un deuxième réglage de puissance défini par l'utilisateur à utiliser en association avec le deuxième capteur d'activation ; et
un circuit de commande d'alimentation (22) configuré pour commander l'alimentation d'un vaporisateur (48) pour générer de la vapeur à partir d'un matériau précurseur de vapeur pour inhalation par l'utilisateur, le circuit de commande d'alimentation étant configuré pour commander l'alimentation du vaporisateur en fonction du premier réglage de puissance défini par l'utilisateur en réponse à la détection d'une activation par l'utilisateur du premier capteur d'activation et pour commander l'alimentation du vaporisateur en fonction du deuxième réglage de puissance défini par l'utilisateur en réponse à la détection d'une activation par l'utilisateur du deuxième capteur d'activation.

2. Système de délivrance de vapeur de la revendication 1, dans lequel le premier capteur d'activation est configuré pour détecter une activation par l'utilisateur en réponse à un appui par l'utilisateur sur un premier bouton du système de délivrance de vapeur et le deuxième capteur d'activation est configuré pour détecter une activation par l'utilisateur en réponse à un appui par l'utilisateur sur un deuxième bouton du système de délivrance de vapeur.

3. Système de délivrance de vapeur de la revendication 1, dans lequel le premier capteur d'activation est configuré pour détecter une activation par l'utilisateur en réponse à un appui par l'utilisateur sur un bouton du système de délivrance de vapeur en fonction d'une première séquence d'appui prédéfinie et le deuxième capteur d'activation est configuré pour détecter une activation par l'utilisateur en réponse à un appui par l'utilisateur sur le bouton en fonction d'une deuxième séquence d'appui prédéfinie.

4. Système de délivrance de vapeur de la revendication 3, dans lequel la première séquence d'appui et/ou la deuxième séquence d'appui correspondent à l'appui par l'utilisateur sur le bouton un nombre prédéfini de fois dans un temps prédéfini.

5. Système de délivrance de vapeur de la revendication 1, dans lequel le premier capteur d'activation est configuré pour détecter une activation par l'utilisateur en réponse à une inhalation par l'utilisateur sur le système de délivrance de vapeur.

6. Système de délivrance de vapeur de l'une quelconque des revendications 1 à 5, dans lequel le circuit de commande d'alimentation est configuré pour commander l'alimentation du vaporisateur en fonction du deuxième réglage de puissance défini par l'utilisateur uniquement quand une activation par l'utilisateur du deuxième capteur d'activation est détectée conjointement avec une activation par l'utilisateur du premier capteur d'activation.

7. Système de délivrance de vapeur de l'une quelconque des revendications 1 à 6, dans lequel le circuit de commande d'alimentation est configuré pour commander l'alimentation du vaporisateur en fonction d'un troisième réglage de puissance en réponse à la détection d'une activation simultanée par l'utilisateur du premier capteur d'activation et du deuxième capteur d'activation.

8. Système de délivrance de vapeur de la revendication 7, dans lequel le troisième réglage de puissance est un réglage de puissance défini par l'utilisateur et le circuit de programmation utilisateur est configuré, en réponse à une entrée utilisateur, pour recevoir et stocker le troisième réglage de puissance défini par l'utilisateur.

9. Système de délivrance de vapeur de l'une quelconque des revendications 1 à 8, dans lequel le circuit de programmation utilisateur est configuré pour recevoir une entrée utilisateur par le même mécanisme d'entrée utilisateur que le premier capteur d'activation et/ou le deuxième capteur d'activation.

10. Système de délivrance de vapeur de l'une quelconque des revendications 1 à 9, comprenant en outre un écran (24) destiné à fournir une indication du premier réglage de puissance défini par l'utilisateur et/ou une indication du deuxième réglage de puissance défini par l'utilisateur.

11. Système de délivrance de vapeur de l'une quelconque des revendications 1 à 10, comprenant en outre un autre capteur d'activation, et dans lequel le circuit de programmation utilisateur est également configuré, en réponse à une entrée utilisateur, pour recevoir et stocker un autre réglage de puissance défini par l'utilisateur à utiliser en association avec l'autre capteur d'activation, et le circuit de commande d'alimentation est configuré pour commander l'alimentation du vaporisateur en fonction de l'autre réglage de puissance défini par l'utilisateur en réponse à la détection d'une activation par l'utilisateur de l'autre capteur d'activation.

12. Système de délivrance de vapeur de l'une quelconque des revendications 1 à 11, le système de délivrance de vapeur comprenant en outre le vaporisateur.

13. Système de délivrance de vapeur de l'une quelconque des revendications 1 à 12, le système de délivrance de vapeur comprenant en outre le matériau précurseur de vapeur.

14. Système de délivrance de vapeur de l'une quelconque des revendications 1 à 13, le système de délivrance de vapeur étant formé à partir d'une partie réutilisable (2) comprenant le premier capteur d'activation, le deuxième capteur d'activation, le circuit de programmation utilisateur, le circuit de commande d'alimentation et une alimentation, et une partie cartouche (4) comprenant le matériau précurseur de vapeur, la partie cartouche pouvant être couplée de façon amovible à la partie réutilisable pour utilisation.

15. Procédé non thérapeutique de fonctionnement d'un système de délivrance de vapeur (1), comprenant la réception d'une entrée utilisateur pour indiquer un premier réglage de puissance défini par l'utilisateur à utiliser en association avec un premier capteur d'activation (14) du système de délivrance de vapeur et un deuxième réglage de puissance défini par l'utilisateur à utiliser en association avec un deuxième capteur d'activation (16) du système de délivrance de vapeur et, par la suite, alimenter un vaporisateur (48) pour générer de la vapeur à partir d'un matériau précurseur de vapeur pour inhalation par l'utilisateur en fonction du premier réglage de puissance défini par l'utilisateur en réponse à la détection d'une activation par l'utilisateur du premier capteur d'activation et l'alimentation du vaporisateur pour générer de la vapeur à partir du matériau précurseur de vapeur pour inhalation par l'utilisateur en fonction du deuxième réglage de puissance défini par l'utilisateur en réponse à la détection d'une activation par l'utilisateur du deuxième capteur d'activation.
